# EUROPEAN PATENT APPLICATION

(11) **EP 2 275 101 A1**
(43) Date of publication of application: **19.01.2011**
(21) Application number: 10176352.2
(22) Date of filing: 14.05.2002
(51) Int. Cl.: A61K 31/22, A23L 1/30, A23K 1/16

(54) **Polar lipid-rich fraction containing stearidonic acid and gamma linolenic acid from plant seeds and microbes, and its uses**

(30) Priority: 14.05.2001 US 291484 P
(62) Divisional of application: 02747838.7
(71) Applicant: MARTEK BIOSCIENCES CORPORATION, Columbia, MD 21045 (US)
(72) Inventor: The designation of the inventor has not yet been filed
(74) Representative: Mallalieu, Catherine Louise

(57) **Abstract**

The production and use, and in particular, the extraction, separation, synthesis and recover of polar lipid-rich fractions containing gamma linolenic acid (GLA) and/or stearidonic acid (SDA) from seeds and microorganisms and their use in human food applications, animal feed, pharmaceuticals and cosmetics.

## Description

### FIELD OF THE INVENTION

The present invention relates to the fields of production and use, and in particular, the extraction, separation, synthesis and recovery of polar lipid-rich fractions containing gamma linolenic acid (GLA) and/or stearidonic acid (SDA) from seeds and microorganisms and their use in human food applications, animal feed, pharmaceuticals and cosmetics.

### BACKGROUND OF THE INVENTION

Polyunsaturated fatty acids of the omega-6 and omega-3 series represent a special class of bioactive lipids in that they are important structurally in membranes in the body, but also participate directly and indirectly in communication between cells through the eicosanoid pathways and by their influence of these fatty acids on gene expression. Two of these fatty acids GLA (gammalinolenic acid; C18:3n-6) and SDA (stearidonic acid; C18:4n-3) have been shown to be effective in treating inflammatory conditions, autoimmune conditions, women's health conditions (e.g. menopause and premenstrual disorders) and fatty acid imbalances in infants and animals. Recent evidence indicates that some polyunsaturated fatty acids may be more bioavailable when supplied in a phospholipid form than in a triglyceride form. GLA and SDA have historically been supplied to the nutritional supplement markets in the form of oil extracted from seeds. However recent evidence indicates that some polyunsaturated fatty acids may be more bioavailable in a phospholipid form rather than in a triglyceride form. This may be because of the bipolar nature of phospholipids making them readily solubilized in the gut and available for digestion and uptake. This same bipolar property of phospholipids additionally would make these fatty acids, such as GLA and SDA, more functional in topical applications such as creams and lotions because of their ability to participate in emulsification processes. The present inventors propose that there may be important advantages in supplying GLA and SDA in the form of phospholipids and improved processes for recovering polar lipids enriched in these fatty acids are also needed.

Examples of polar lipids include phospholipids (e.g. phosphatidylcholine, phosphatidylethanolamine, phosphatidylinositol, phosphatidylserine, phosphatidylglycerol, diphosphatidylglycerols), cephalins, sphingolipids (sphingomyelins and glycosphingolipids), and glycoglycerolipids. Phospholipids are composed of the following major structural units: fatty acids, glycerol, phosphoric acid, and amino alcohols. They are generally considered to be structural lipids, playing important roles in the structure of the membranes of plants, microbes and animals. Because of their chemical structure, polar lipids exhibit a bipolar nature, exhibiting solubility or partial solubility in both polar and non-polar solvents. The term polar lipid within the present description is not limited to natural polar lipids but also includes chemically modified polar lipids. Although the term oil has various meanings, as used herein, it will refer to the triacylglycerol fraction.

One of the important characteristics of polar lipids, and especially phospholipids, is that they commonly contain polyunsaturated fatty acids (PUFAs: fatty acids with 2 or more unsaturated bonds). In many plant, microbial and animal systems, they are especially enriched in the highly unsaturated fatty acids (HUFAs: fatty acids with 4 or more unsaturated bonds) of the omega-3 and omega-6 series. Although these highly unsaturated fatty acids are considered unstable in triacylglycerol form, they exhibit enhanced stability when incorporated in phospholipids.

The primary sources of commercial PUFA-rich phospholipids are soybeans and canola seeds. These biomaterials do not contain any appreciable amounts of GLA or SDA unless they have been genetically modified. The phospholipids (commonly called lecithins) are routinely recovered from these oilseeds as a by-product of the vegetable oil extraction process. For example, in the production of soybean or canola oil, the beans (seeds) are first heat-treated and then crushed, ground, and/or flaked, followed by extraction with a non-polar solvent such as hexane. Hexane removes the triacylglycerol-rich fraction from the seeds together with a varying amount of polar lipids (lecithins). The extracted oil is then de-gummed (lecithin removal) either physically or chemically as a part of the normal oil refining process and the precipitated lecithins recovered. This process however has two disadvantages: (1) the seeds must be heat-treated before extraction with hexane, both increasing the processing cost and denaturing the protein fraction, thereby decreasing its value as a by-product; and (2) the use of the non-polar solvents such as hexane also presents toxicity and flammability problems that must be dealt with.

The crude lecithin extracted in the "de-gumming" process can contain up to about 33% oil (triacylglycerols) along with sterols and glucosides. One preferred method for separating this oil from the crude lecithin is by extraction with acetone. The oil (triacylglycerols) is soluble in acetone and the lecithin is not. The acetone solution is separated from the precipitate (lecithin) by centrifugation and the precipitate dried under first a fluidized bed drier and then a vacuum drying oven to recover the residual acetone as the product is dried. Drying temperatures of 50-70°C are commonly used. The resulting dried lecithins contain approximately 2-4% by weight of oil (triacylglycerols). Process temperatures above 70°C can lead to thermal decomposition of the phospholipids. However, even at temperatures below 70°C the presence of acetone leads to the formation of products that can impair the organoleptic quality of the phospholipids. These byproducts can impart musty odors to the product and also a pungent aftertaste.

What is needed is an improved process for effectively recovering polar lipids and phospholipids rich in GLA and SDA from biomaterials that enables the use of these fatty acid in food, nutritional supplement, pharmaceutical and cosmetic applications. Furthermore the fractions are needed as an ingredient in feed for companion animals and in aquaculture.

### SUMMARY OF THE INVENTION

In accordance with the present invention, an improved process is provided for recovering polar lipids enriched in gamma linolenic acid (GLA) and/or stearidonic acid (SDA) from native biomaterials such as seeds and microorganisms and the use thereof.

In one embodiment of the present invention, a method is provided for providing a human, animal or aquaculture organism diet supplement enriched with at least one of gamma linolenic acid (GLA) and stearidonic acid (SDA). The method includes the steps of producing a GLA- and/or SDA-enriched polar lipid-rich fraction from seeds or microbes; and providing the GLA- and/or SDA-enriched polar lipid-rich fraction in a form consumable by humans and animals. Preferably, the animals are companion animals.

In another embodiment of the present invention, a method is provided for treating a deficiency in at least one of gamma linolenic acid (GLA) and stearidonic acid (SDA). The method includes the steps of producing a GLA- and/or SDA-enriched polar lipid-rich fraction from seeds or microbes; and providing the GLA- and/or SDA-enriched polar lipid-rich fraction to treat the deficiency. The deficiency can lead to an inflammatory condition, an autoimmune condition, a woman's health condition or an infant's health condition.

In another embodiment of the present invention, a method is provided for treating chronic inflammatory disease states of the lung, including but not limited to chronic obstructive pulmonary disease (COPD), asthma and cystic fibrosis. The method includes the steps of producing a GLA- and/or SDA-enriched purified phospholipid fraction from seeds or microbes; blending the GLA- and/or SDA-rich phospholipid fraction with at least one of EPA-, GLA- or SDA-rich oils; and producing an aerosol, such as by providing an aerosol delivery system, for the treatment of the disease states.

In another embodiment of the present invention, a method is provided for the treatment of skin lesions, induced burn, UV-irradiation or other skin disorders. The method includes the steps of producing a GLA- and/or SDA-enriched purified phospholipid fraction from seeds or microbes; blending the GLA- and/or SDA-rich phospholipid fraction with at least one EPA-, GLA- or SDA-rich oil; and producing a lotion or cream for the treatment of the skin disorders.

In another embodiment of the present invention, a method is provided for treating cachexia or fat malabsorption. The method includes the steps of producing a GLA- and/or SDA-enriched purified phospholipids; blending the GLA- and/or SDA-rich polar lipid fractions with at least one other purified phospholipid; blending the GLA- and/or SDA-rich polar lipid fractions with at least one DHA, EPA, GLA- or SDA-rich oil; and producing a liquid or dry dietetic product for the treatment of the disease states. The cachexia or fat malabsorption can result from the illnesses such as cancer and Crohn's disease. The at least one other purified phospholipid can be obtained from sources such as soybeans, rapeseed, canola, corn, peanuts, flax seed, linseed, sunflower, safflower, and eggs.

In another embodiment of the present invention, a method is provided for the treatment of *H. pylori*-infection of the gastrointestinal tract. The method includes the steps of producing a GLA- and/or SDA-enriched purified phospholipid fraction from seeds or microbes; blending the GLA- and/or SDA-rich phospholipid fraction with at least one EPA-, GLA- or SDA-rich oil; and producing a fat emulsion or a dietetic product for the treatment of the disease.

In another embodiment of the present invention, a method is provided for providing a fat blend enriched with at least one of gamma linolenic acid (GLA) and stearidonic acid (SDA). The method includes the steps of extracting a GLA- and/or SDA-enriched polar lipid-rich fraction from seeds or microbes; and mixing the GLA- and/or SDA-enriched polar lipid-rich fraction with another oil. Preferably, the another oil is selected from the group consisting of fish oil, microbial oil, vegetable oil, GLA-containing oil, SDA-containing oil and mixtures thereof.

In another embodiment of the present invention, a method is provided for providing a blend of polar lipids enriched with at least one of gamma linolenic acid (GLA) and stearidonic acid (SDA). The method includes the steps of extracting a GLA- and/or SDA-enriched polar lipid-rich fraction from seeds or microbes; and mixing the GLA- and/or SDA-enriched polar lipid-rich fraction with another polar lipid. Preferably, the another polar lipid is selected from the group consisting of soy polar lipids, rapeseed polar lipids, sunflower polar lipids, safflower polar lipids, canola polar lipids, linseed polar lipids, flaxseed polar lipids, peanut polar lipids, egg yolk polar lipids and mixtures thereof.

In another embodiment of the present invention, a fat blend is provided that is enriched with at least one of gamma linolenic acid (GLA) and stearidonic acid (SDA) comprising a GLA- and/or SDA-enriched polar lipid-rich fraction from seeds or microbes; and another oil. Preferably, the another oil is selected from the group consisting of fish oil, microbial oil, vegetable oil, GLA-containing oil, SDA-containing oil and mixtures thereof.

In another embodiment of the present invention, a method is provided for providing a blend of polar lipids enriched with at least one of gamma linolenic acid (GLA) and stearidonic acid (SDA). The method includes the steps of extracting a GLA- and/or SDA-enriched polar lipid-rich fraction from seeds or microbes; and mixing the GLA- and/or SDA-enriched polar lipid-rich fraction with another polar lipid. Preferably, the another polar lipid is selected from the group consisting of soy polar lipids, rape seed polar lipids, sunflower polar lipids, safflower polar lipids, canola polar lipids, linseed polar lipids, flaxseed polar lipids, peanut polar lipids, egg yolk polar lipids and mixtures thereof.

In another embodiment of the present invention, purified phospholipids enriched with at least one gamma linolenic acid (GLA) and stearidonic acid (SDA) derived from polar lipid-rich fraction extracted from seeds or microbes are provided. Preferably, the GLA- and/or SDA-enriched phospholipid-fraction is in a form consumable by humans and animals.

Preferably, polar lipid-rich fractions of the methods or products of the present invention can be used as an ingredient of dietetic, pharmaceutical and cosmetic applications.

As used herein, the term dietetic includes nutritional supplements (in gel-cap, tablet, liquid, emulsion, powder or any other form) and food. The term pharmaceutical includes all compounds ingested (including special enteral and parenteral nutrition products) or injected or received intravenously, for the treatment of diseases or metabolic imbalances.

Preferably, fat blends of the methods or products of the present invention can be used as an ingredient of dietetic, pharmaceutical and cosmetic applications.

Preferably, blends of polar lipids of the methods or products of the present invention can be used as an ingredient of dietetic, pharmaceutical or cosmetic applications.

Preferably, purified phospholipids of the methods or products of the present invention can be used as an ingredient of dietetic, pharmaceutical or cosmetic applications.

Preferably, seeds useful in the methods and products of the present invention are from the plant families Boraginaceae, Onagraceae, Saxifragaceae, Scrophulariaceae or Cannabaceae, and more preferably, the seeds are selected from the group consisting of borage, echium, evening primrose and black currant.

Preferably, the microbes useful in the methods and products of the present invention are selected from fungi, microalgae and bacteria. More preferably, the microbes are selected from the group of genera consisting of *Mortierella, Mucor, Blastocladiella, Choanephora, Conidiobolus, Entomophthora, Helicostylum, Phycomyces, Rhizopus, Beauveria,* and *Pythium.*

Preferably, the GLA of the products and methods of the present invention makes up at least two weight percent of the total fatty acids of the polar lipid fraction.

Preferably, the SDA of the products and methods of the present invention makes up at least two weight percent of the total fatty acids of the polar lipid fraction.

Preferably, the plant seeds of the products and methods of the present invention have been genetically modified, and more preferably, the seeds have been genetically modified to increase the production of at least one of SDA and GLA.

Preferably, the seeds of the methods and products of the present invention are selected from the group consisting of canola, rapeseed, linseed, flaxseed, sunflower, safflower, soybeans, peanuts and corn.

Preferably, the polar lipid-rich fraction is extracted from the seeds or microbes using alcohol.

In an alternative embodiment of the present invention, the polar lipid-rich fraction is derived as a by-product of oil extraction, e.g. by de-gumming, from the seeds or microbes using hexane and other nonpolar solvents.

Preferably, the polar lipid-rich fraction is extracted from the seeds or microbes by use of gravity or centrifugal extraction technology.

### DETAILED DESCRIPTION OF THE INVENTION

Because of their bipolar nature, polar lipids (including phospholipids) are of significant commercial interest as wetting and emulsifying agents. These properties may also help make PUFAs in the phospholipids more bioavailable, in addition to enhancing their stability. These properties make phospholipids ideal forms of ingredients for use in nutritional supplements, food, infant formula, pharmaceutical, and cosmetic applications. Dietary benefits of phospholipids include both improved absorption and improved incorporation. Phospholipids also have a broad range of functionality in the body in that they are important cell membrane constituents, they are good emulsifiers, they can act as intestinal surfactants, serve as a choline source and as a source of PUFAs.

GLA and SDA are normally produced for the nutritional supplement market through hexane extraction of seeds from the plant families Boraginaceae, Onagraceae, Saxifragaceae, Scrophulariaceae or Cannabaceae. These families include borage, echium, evening primrose and black currant. The phospholipids are removed in a degumming step that produces a waste material comprising a complex mixture of neutral lipids, sterols, glucosides and phospholipids. This material is normally sold to the domestic animal feed industry to dispose of it. To the best of the present inventors' knowledge, there are no phospholipid forms of GLA and/or SDA available in the nutritional supplement, food, companion animal or aquaculture markets.

Besides oilseeds, there are also microbial sources of SDA and GLA but none of these is commercially available. Microorganisms known to contain GLA and/or SDA are found in yeast and the following genera of fungi: *Mortierella, Mucor, Blastocladiella, Choanephora, Conidiobolus, Entomophthora, Helicostylum, Rhizopus, Beauveria, Thamnidium, Lactarius, Cantherellus, Polyporus, Glomus, Zygorhynchus,* and *Pythium;* and genera of algae and algae-like microorganisms including: *Chlorella, Cyanidium, Scenedesmus, Chlamydomonas, Ankistrodesmus, Enteromorpha, Oocystis, Dunaliella, Heteromastix, Ochromonas, Prymnesium, Isochiysis, Dicrateria, Fucus, Gonlaulux, Amphidinium, Peridinium, Hemiselmis, Cryptomonas, Chroomonas, Rhodomonas, Hemiselmis, Thraustochytrium,* and *Schizochytium.* For the purposes of this application members of the former thraustochytrid genus *Ulkenia* are considered to be part of the genus *Thraustochytrium.* Microorganisms are good sources of phospholipids because they can be grown in culture in a manner that optimizes phospholipid production and minimizes triglyceride (oil) production. On the other hand the methods used in this invention allow both oil and phospholipids to be recovered separately in forms that can be used directly in food, feed, nutritional supplements, cosmetic or pharmaceutical application.

GLA and SDA phospholipids can be recovered from oilseeds through the degumming process described above. However, as noted, this produces a complex material containing many other compounds including neutral lipids, sterols, glucosides, etc

A preferred embodiment of the present invention is to use alcohol and centrifugation to recover the GLA- and SDA-rich phospholipids. Preferred methods for this recovery are described in the following references which are incorporated by reference herein in their entirety:
i. PCT Application Serial No. PCT/US01/12047, entitled "Method for the Fractionation of Oil and Polar Lipid-Containing Native Raw Materials" filed April 12, 2001;
ii. PCT Application Serial No. PCT/US01/12049, entitled "Method For The Fractionation Of Oil And Polar Lipid-Containing Native Raw Materials Using Water-Soluble Organic Solvent And Centrifugation" filed April 12, 2001.

Although these are preferred extraction methods, any suitable extraction method can be employed with the present invention. Once the GLA- and SDA-rich phospholipids fractions have been extracted by these preferred processes, they can be used directly as ingredients or they can be purified further and even separated into phospholipid classes by well-known techniques such as different forms of chromatography, molecular distillation, and special refining techniques. The phospholipid rich polar lipids or the purified phospholipid rich fractions can also be mixed with another lipid or oil such as fish lipids, microbial lipids, vegetable lipids, GLA-containing lipids, SDA-containing lipids and mixtures thereof, or be mixed with another phospholipid fraction (lecithin) such as soy or egg yolk lecithin, sunflower lecithin, peanut lecithin or mixtures thereof prior to use as a nutritional supplement, feed or food ingredient. These mixtures of phospholipids can also be incorporated into creams or lotions for topical applications (e.g. treating of skin conditions) or skin lesions induced by burns, UV-irradiation or other skin damaging processes. The mixtures can also be processed to produce a liquid or spray-dried dietetic product or fat emulsion for treating cachexia and severe fat malabsorption or for treatment of *H. pylori* infection of the gastrointestinal tract, or be used to produce an aerosol (spray) for the treatment of chronic inflammatory disease states of the lung (COPD, asthma, cystic fibrosis).

Advantages of the present invention including providing GLA and SDA in a more bioactive and functional form (phospholipid) than the triglyceride form and include a better process: a) no need for heat treatment; b) no use of toxic solvents (like hexane) and c) no artifacts and off-flavors due to the use of acetone) for recovering these phospholipids from oilseeds and microbes.

The following example is provided for the purpose of illustration and are not intended to limit the scope of the present invention.

### EXAMPLE

### Example 1

Phospholipids were extracted from four types of oilseeds and the total fatty acid content of the phospholipids was determined by gas chromatography. The results are presented in Table 1. As can be observed the phospholipid fraction of these seeds can be used to deliver GLA and or SDA and in this form these bioactive fatty acids should be more stable, more bioavailable, and more functional.

**Table 1. Total fatty acid content of phospholipids extracted from four types of oilseeds containing GLA and SDA.**

| COMPOUND | | **Black Currant PL's** | **Borage PL's** | **EPO PL's** | **Echium PL's** |
|---|---|---|---|---|---|
| | | % TFA | % TFA | % TFA | % TFA |
| MYRISTATE | C14:0 | 0,56 | 0,67 | 0,61 | 1,12 |
| MYRISTOLEATE | C14:1 | 0,00 | 0,00 | 0,00 | 0,00 |
| PALMITATE | C16:0 | 23,84 | 21,71 | 17,78 | 23,01 |
| PALMITOLEATE | C16:1 | 0,43 | 0,00 | 0,46 | 1,75 |
| STEARATE | C18:0 | 4,59 | 8,55 | 7,79 | 5,69 |
| OLEATE | C18:1 | 18,70 | 25,52 | 27,67 | 24,56 |
| LINOLEATE | C18:2n6 | 36,20 | 30,89 | 37,92 | 17,75 |
| GAMMA LINOLENATE | C18:3n6 | 5,02 | 7,26 | 2,33 | 7,17 |
| ARACHIDATE | C20:0 | 0,51 | 1,27 | 1,20 | 0,00 |
| LINOLENATE | C18:3n3 | 7,30 | 4,15 | 1,00 | 14,09 |
| OCTADECATETRAENOATE | C18:4 | 1,20 | 0,00 | 0,23 | 4,86 |
| EICOSENOATE-11 | C20:1 | 0,99 | 0,00 | 0,60 | 0,00 |
| EICOSADIENOATE-11,14 | C20:2 | 0,00 | 0,00 | 0,00 | 0,00 |
| BEHENATE | C22:0 | 0,66 | 0,00 | 1,68 | 0,00 |
| EICOSATRIENOATE | C20:3n3 | 0,00 | 0,00 | 0,00 | 0,00 |
| ARACHIDONATE | C20:4 n6 | 0,00 | 0,00 | 0,00 | 0,00 |
| ERUCATE | C22:1 | 0,00 | 0,00 | 0,00 | 0,00 |
| EICOSAPENTAENOATE | C20:5n3 | 0,00 | 0,00 | 0,00 | 0,00 |
| LIGNOCERATE | C24:0 | 0,00 | 0,00 | 0,72 | 0,00 |
| NERVONATE | C24:1 | 0,00 | 0,00 | 0,00 | 0,00 |
| DOCOSAPENTAENOATE n-6 | C22:5n6 | 0,00 | 0,00 | 0,00 | 0,00 |
| DOCOSAPENTAENOATE n-3 | C22:5n3 | 0,00 | 0,00 | 0,00 | 0,00 |
| DOCOSAHEXAENOATE | C22:6n3 | 0,00 | 0,00 | 0,00 | 0,00 |
| | | 100,00 | 100,00 | 100,00 | 100,00 |

The present invention, in various embodiments, includes components, methods, processes, systems and/or apparatus substantially as depicted and described herein, including various embodiments, subcombinations, and subsets thereof. Those of skill in the art will understand how to make and use the present invention after understanding the present disclosure. The present invention, in various embodiments, includes providing devices and processes in the absence of items not depicted and/or described herein or in various embodiments hereof, including in the absence of such items as may have been used in previous devices or processes, *e*.*g*., for improving performance, achieving ease and/or reducing cost of implementation.

The foregoing discussion of the invention has been presented for purposes of illustration and description. The foregoing is not intended to limit the invention to the form or forms disclosed herein. Although the description of the invention has included description of one or more embodiments and certain variations and modifications, other variations and modifications are within the scope of the invention, e.g., as may be within the skill and knowledge of those in the art, after understanding the present disclosure. It is intended to obtain rights which include alternative embodiments to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

Various preferred features and embodiments of the present invention will now be described with reference to the following numbered paragraphs (paras).
1. A method for providing a human, animal or aquaculture organism diet supplement enriched with at least one of gamma linolenic acid (GLA) and stearidonic acid (SDA) comprising the steps:
   (a) producing a GLA- and/or SDA-enriched polar lipid-rich fraction from seeds or microbes; and
   (b) providing said GLA- and/or SDA-enriched polar lipid-rich fraction in a form consumable by humans and animals.
2. A method for treating a deficiency in at least one of gamma linolenic acid (GLA) and stearidonic acid (SDA) comprising the steps:
   (a) producing a GLA- and/or SDA-enriched polar lipid-rich fraction from seeds or microbes; and
   (b) providing said GLA- and/or SDA-enriched polar lipid-rich fraction to treat said deficiency.
3. The method of para 2, wherein said deficiency results in an inflammatory condition, an auto-immune condition, a woman's health condition or an infant's health condition.
4. A method for treating chronic inflammatory disease states of the lung, including but not limited to COPD, asthma and cystic fibrosis, comprising the steps:
   (a) producing a GLA- and/or SDA-enriched purified phospholipid fraction from seeds or microbes;
   (b) blending said GLA- and/or SDA-rich phospholipid fraction with at least one of EPA-, GLA- or SDA-rich oils; and
   (c) producing an aerosol for the treatment of said disease states
5. A method for the treatment of skin lesions, induced bum, UV-irradiation or other skin disorders comprising the steps:
   (a) producing a GLA- and/or SDA-enriched purified phospholipid fraction from seeds or microbes;
   (b) blending said GLA- and/or SDA-rich phospholipid fraction with at least one EPA-, GLA- or SDA-rich oil; and
   (c) producing a lotion or creme for the treatment of said skin disorders.
6. A method for treating cachexia or fat malabsorption comprising the steps:
   (a) producing a GLA- and/or SDA-enriched purified phospholipids;
   (b) blending said GLA- and/or SDA-rich polar lipid fractions with at least one other purified phospholipid;
   (c) blending said GLA- and/or SDA-rich polar lipid fractions with at least one DHA, EPA, GLA- or SDA-rich oil; and
   (d) producing a liquid or dry dietetic product for the treatment of said disease states.
7. A method for the treatment of *H*. *pylori*-infection of the gastrointestinal tract comprising the steps:
   (a) producing a GLA- and/or SDA-enriched purified phospholipid fraction from seeds or microbes;
   (b) blending said GLA- and/or SDA-rich phospholipid fraction with at least one EPA-, GLA- or SDA-rich oil; and
   (c) producing a fat emulsion or a dietetic product for the treatment of said disease.
8. A method for providing a fat blend enriched with at least one of gamma linolenic acid (GLA) and stearidonic acid (SDA) comprising the steps:
   (a) extracting a GLA- and/or SDA-enriched polar lipid-rich fraction from seeds or microbes; and
   (b) mixing said GLA- and/or SDA-enriched polar lipid-rich fraction with another oil.
9. The method of para 8, wherein said another oil is selected from the group consisting of fish oil, microbial oil, vegetable oil, GLA-containing oil, SDA-containing oil and mixtures thereof.
10. A method for providing a blend of polar lipids enriched with at least one of gamma linolenic acid (GLA) and stearidonic acid (SDA) comprising the steps:
   (a) extracting a GLA- and/or SDA-enriched polar lipid-rich fraction from seeds or microbes; and
   (b) mixing said GLA- and/or SDA-enriched polar lipid-rich fraction with another polar lipid.
11. The method of para 10, wherein said another polar lipid is selected from the group consisting of soy polar lipids, rapeseed polar lipids, sunflower polar lipids, safflower polar lipids, canola polar lipids, linseed polar lipids, flaxseed polar lipids, peanut polar lipids, egg yolk polar lipids and mixtures thereof.
12. A fat blend enriched with at least one of gamma linolenic acid (GLA) and stearidonic acid (SDA) comprising:
   (a) a GLA- and/or SDA-enriched polar lipid-rich fraction from seeds or microbes; and
   (b) another oil.
13. The oil of para 12, wherein said another oil is selected from the group consisting of fish oil, microbial oil, vegetable oil, GLA-containing oil, SDA-containing oil and mixtures thereof.
14. A blend of polar lipids enriched with at least one of gamma linolenic acid (GLA) and stearidonic acid (SDA) comprising the steps:
   (a) extracting a GLA- and/or SDA-enriched polar lipid-rich fraction from seeds or microbes; and
   (b) mixing said GLA- and/or SDA-enriched polar lipid-rich fraction with another polar lipid.
15. The method of para 14, wherein said another polar lipid is selected from the group consisting of soy polar lipids, rape seed polar lipids, sunflower polar lipids, safflower polar lipids, canola polar lipids, linseed polar lipids, flaxseed polar lipids, peanut polar lipids, egg yolk polar lipids and mixtures thereof.
16. Purified phospholipids enriched with at least one gamma linolenic acid (GLA) and stearidonic acid (SDA) derived from polar lipid-rich fraction extracted from seeds or microbes.
17. The purified phospholipids of para 16, wherein said GLA- and/or SDA-enriched phospholipid-fraction is in a form consumable by humans and animals.
18. A polar lipid-rich fraction of the method or product of any preceding para as an ingredient of dietetic, pharmaceutical and cosmetic applications.
19. A fat blend of the method or product of any preceding para as an ingredient of dietetic, pharmaceutical and cosmetic applications.
20. A blend of polar lipids of the method or product of any preceding para as an ingredient of dietetic, pharmaceutical or cosmetic applications.
21. The purified phospholipids of the method or product of any preceding para as an ingredient of dietetic, pharmaceutical or cosmetic applications.
22. The method or product of any preceding para, wherein said seeds are from the plant families Boraginaceae, Onagraceae, Saxifragaceae, Scrophulariaceae or Cannabaceae.
23. The method or product of any preceding para, wherein said seeds are selected from the group consisting of borage, echium, evening primrose and black currant.
24. The method or product of any preceding para, wherein said microbes are selected from fungi, microalgae and bacteria.
25. The method or product of any preceding para, wherein said microbes are selected from the group of genera consisting of *Mortierella, Mucor, Blastocladiella, Choanephora, Conidiobolus, Entomophthora, Helicostylum, Phycomyces, Rhizopus, Beauveria,* and *Pythium.*
26. The method of or product of any preceding para, wherein GLA comprises at least two weight percent of the total fatty acids of the polar lipid fraction.
27. The method or product of any preceding para, wherein SDA comprises at least two weight percent of the total fatty acids of the polar lipid fraction.
28. The method or product of any preceding para, wherein said plant seeds have been genetically modified.
29. The method or product of any preceding para, wherein said seeds have been genetically modified to increase the production of at least one of SDA and GLA.
30. The method or product of any preceding para, wherein said seeds are selected from the group consisting of canola, rapeseed, linseed, flaxseed, sunflower, safflower, soybeans, peanuts and corn.
31. The method or product of any preceding para, wherein said polar lipid-rich fraction is extracted from said seeds or microbes using alcohol.
32. The method or product of any preceding para, wherein said polar lipid-rich fraction is derived as a by-product of oil extraction from said seeds or microbes using hexane and other nonpolar solvents.
33. The method or product of any preceding para, wherein said polar lipid-rich fraction is extracted from said seeds or microbes by use of gravity or centrifugal extraction technology.

## Claims

1. A gamma linolenic acid (GLA)- and/or stearidonic acid (SDA)-enriched polar lipid-rich fraction extracted from seeds or microbes.

2. The polar lipid-rich fraction of claim 1, wherein GLA comprises from 1% to 7.5% of the total fatty acids present in the fraction.

3. The polar lipid-rich fraction of claim 1 or 2, wherein SDA comprises from 0.2% to 5% of the total fatty acids present in the fraction.

4. The polar lipid-rich fraction of any one of claims 1 to 3, wherein said seeds are from the plant families Boraginaceae, Onagraceae, Saxifragaceae, Scrophulariaceae, or Cannabaceae.

5. The polar lipid-rich fraction of any one of claims 1 to 3, wherein said seeds have been genetically modified to increase the production of at least one of SDA and GLA.

6. The polar lipid-rich fraction of any one of any preceding claim, wherein said seeds are selected from the group consisting of canola, rapeseed, linseed, flaxseed, sunflower, safflower, soybeans, peanuts, and corn.

7. The polar lipid-rich fraction of any one of claims 1 to 3, wherein said microbes are selected from fungi, microalgae, and bacteria.

8. The polar lipid-rich fraction of any one of claims 1 to 3, wherein said microbes are selected from the group of genera consisting of *Mortierella, Mucor, Blastocladiella, Choanephora, Conidiobolus, Entomophthora, Helicostylum, Phycomyces, Rhizopus, Beauveria,* and *Pythium.*

9. The polar lipid-rich fraction of any preceding claim, wherein said polar lipid-rich fraction is extracted from said seeds or microbes using alcohol.

10. The polar lipid-rich fraction of any one of claims 1 to 8, wherein said polar lipid-rich fraction is derived as a by product of oil extraction from said seeds or microbes using hexane and other non-polar solvents.

11. The polar lipid-rich fraction of any one of claims 1 to 8, wherein said polar lipid-rich fraction is extracted from said seeds or microbes by use of gravity or centrifugal extraction technology.

12. A fat blend enriched with at least one of GLA and SDA, comprising:
(a) the polar lipid-rich fraction of any one of claims 1 to 11; and
(b) another oil.

13. The fat blend of claim 12, wherein said another oil is selected from the group consisting of fish oil, microbial oil, vegetable oil, GLA-containing oil, SDA-containing oil, and mixtures thereof.

14. A polar lipid-rich fraction or fat blend of any preceding claim for treating a deficiency in at least one of GLA and SDA.

15. The polar lipid-rich fraction or fat blend of claim 14, wherein said deficiency results in an inflammatory condition, an auto-immune condition, a woman's health condition, or an infant's health condition.

16. A polar lipid-rich fraction or fat blend of any one of claims 1 to 13 for treating a condition selected from the group consisting of: chronic inflammatory disease states of the lung, skin lesions, induced bum, UV-irradiation or other skin disorders, cachexia, fat malabsorption, and *H. pylori* infection of the gastrointestinal tract.

17. The polar lipid-rich fraction or fat blend of claim 16, wherein the chronic inflammatory disease state of the lung is COPD, asthma, or cystic fibrosis.
